# EUROPEAN PATENT SPECIFICATION

(11) **EP 2 419 728 B1**
(45) Date of publication and mention of the grant of the patent: **08.01.2014**
(21) Application number: 10719285.8
(22) Date of filing: 12.04.2010
(51) Int. Cl.: G01N 33/50

(54) **BIOMARKER FOR MONITORING PATIENTS**
BIOMARKER FÜR PATIENTENÜBERWACHUNG
BIOMARQUEUR DE SURVEILLANCE DES PATIENTS

(30) Priority: 17.04.2009 EP 09305328
(43) Date of publication of application: 22.02.2012
(73) Proprietor: Transgene SA, 67405 Illkirch Graffenstaden Cedex (FR)
(72) Inventor: ACRES, Bruce, F-67000 Strasbourg (FR)
(74) Representative: Vossius & Partner
(86) International application number: PCT/EP2010/054743
(87) International publication number: WO 2010/119003

(56) References cited:
- ROSSI GABRIELA R ET AL: "Effective treatment of preexisting melanoma with whole cell vaccines expressing alpha(1,3)-galactosyl epitopes" CANCER RESEARCH, vol. 65, no. 22, November 2005 (2005-11), pages 10555-10561, XP002589947 ISSN: 0008-5472
- NEIL M AMPEL ET AL: "An Archived Lot of Coccidioidin Induces Specific Coccidioidal Delayed-type Hypersensitivity and Correlates with in vitro Assays of Coccidioidal Cellular Immune Response" MYCOPATHOLOGIA, KLUWER ACADEMIC PUBLISHERS, DO, vol. 161, no. 2, 1 February 2006 (2006-02-01), pages 67-72, XP019259983 ISSN: 1573-0832
- MELICHAR BOHUSLAV ET AL: "The peripheral blood leukocyte phenotype in patients with breast cancer: Effect of doxorubicin/paclitaxel combination chemotherapy" IMMUNOPHARMACOLOGY AND IMMUNOTOXICOLOGY, vol. 23, no. 2, May 2001 (2001-05), pages 163-173, XP008123949 ISSN: 0892-3973
- DEVARAPU SATISH K ET AL: "Triggering of T cell-mediated immune responses by allogenic tumor cell vaccine in patients with oral cancer" IMMUNOPHARMACOLOGY AND IMMUNOTOXICOLOGY, vol. 28, no. 3, July 2006 (2006-07), pages 387-395, XP008110728 ISSN: 0892-3973
- LAVASANI S ET AL: "Monoclonal antibody against T-cell receptor alpha beta induces self-tolerance in chronic experimental autoimmune encephalomyelitis" SCANDINAVIAN JOURNAL OF IMMUNOLOGY, vol. 65, no. 1, January 2007 (2007-01), pages 39-47, XP002589950 ISSN: 0300-9475
- HUANG JING-LONG ET AL: "Reduced expression of CD69 and adhesion molecules of T lymphocytes in asthmatic children receiving immunotherapy." PEDIATRIC ALLERGY AND IMMUNOLOGY : OFFICIAL PUBLICATION OF THE EUROPEAN SOCIETY OF PEDIATRIC ALLERGY AND IMMUNOLOGY DEC 2002 LNKD- PUBMED:12485318, vol. 13, no. 6, December 2002 (2002-12), pages 426-433, XP002589951 ISSN: 0905-6157
- DOBRZANSKI MARK J ET AL: "Tc1 and Tc2 effector cell therapy elicit long-term tumor immunity by contrasting mechanisms that result in complementary endogenous type 1 antitumor responses." JOURNAL OF IMMUNOLOGY, vol. 172, no. 3, 1 February 2004 (2004-02-01), pages 1380-1390, XP002589952 ISSN: 0022-1767

## Description

The present invention is in the field of immunotherapy and relates to methods for determining the efficacy of certain immunotherapy treatments. The methods of the invention include measuring special biomarker at some time following the initiation of immunotherapy treatment to evaluate the clinical outcome of the said treatment. The invention thus has applications to the field of medicine.

Traditional vaccination techniques involving the introduction into an animal system of an antigen (e.g. peptides, proteins) which can induce an immune response, and thereby protect said animal against infection for example, have been known for many years. These techniques have further included the development of both live and inactivated vaccines. Live vaccines are typically attenuated non-pathogenic versions of an infectious agent that are capable of priming an immune response directed against a pathogenic version of the infectious agent.

In recent years there have been advances in the development of recombinant vaccines, especially recombinant live vaccines, in which foreign antigens of interest are encoded and expressed from a vector. Among them, vectors based on recombinant viruses have shown great promise and play an important role in the development of new vaccines. Many viruses have been investigated for their ability to express proteins from foreign pathogens or tumor tissue, and to induce specific immunological responses against these antigens in *vivo.* Generally, these gene-based vaccines can stimulate potent humoral and cellular immune responses and viral vectors may be an effective strategy for both the delivery of antigen-encoding genes and the facilitation and enhancement of antigen presentation. In order to be utilized as a vaccine carrier, the ideal viral vector should be safe and enable efficient presentation of required pathogen-specific antigens to the immune system. Furthermore, the vector system must meet criteria that enable its production on a large-scale basis. Several viral vaccine vectors have thus emerged to date, all of them having relative advantages and limits depending on the proposed application (for a review on recombinant viral vaccines see for example Harrop and Carroll, 2006, Front Biosci., 11, 804-817 ; Yokoyama et al., 1997, J Vet Med Sci.,59, 311-322). The use of the said recombinant vaccines is commonly named targeted immunotherapy or antigen specific and active immunotherapy.

Following the observation in the early 1990's that plasmid DNA vectors could directly transfect animal cells in vivo, significant research efforts have also been undertaken to develop immunotherapy techniques based upon the use of DNA plasmids to induce immune response, by direct introduction into animals of DNA which encodes for antigens. Such techniques which are widely referred as DNA vaccination, or DNA immunotherapy, have now been used to elicit protective immune responses in large number of disease models. For a review on DNA vaccines, see Reyes-Sandoval and Ertl, 2001 (Current Molecular Medicine, 1, 217-243).

A general problem in immunotherapy field however has been the identification of a means of inducing a sufficiently strong immune response in treated individuals to protect against infection and disease.

Therefore there has been for example major effort in recent years, to discover new drug compounds that act by stimulating certain key aspects of the immune system which will serve to increase the immune response induced by immunotherapies. Most of these compounds, referred as immune response modifiers (IRMs) or adjuvants, appear to act through basic immune system mechanisms via Toll-like receptors (TLRs) to induce various important cytokines biosynthesis (e.g. interferons, interleukins, tumor necrosis factor, etc. see for example Schiller et al., 2006, Exp Dermatol., 15, 331-341). Such compounds have been shown to stimulate a rapid release of certain dendritic cell, monocyte/macrophage-derived cytokines and are also capable of stimulating B cells to secrete antibodies which play an important role in the antiviral and antitumor activities of IRM compounds.

Alternatively, immunotherapy strategies have been proposed, most of them being based on a prime-boost vaccination regimen. According to these "prime-boost" immunotherapy protocols, the immune system is first induced by administering to the patient a priming composition and then boosted by administration of a boosting second composition (see for example EP1411974 or US20030191076).

Moreover, it has been shown in the health care context that one treatment can be effective only in specific group of patients. It is thus desirable to provide to physicians tools and methods that will enable them to tailor optimal personalized patient therapies, i.e. to prescribe the right therapy to the right patient at right time, to provide a higher treatment success rate, to monitor the response to the treatment, to increase drug efficacy and safety, to eliminate the unnecessary treatment of patients for whom therapy is not appropriate, to spare the patient unnecessary toxicity and side effects, to reduce the cost to patients and insurers of unnecessary or dangerous ineffective medication, and to improve patient quality of life, eventually making cancer a managed disease, with follow up assays as appropriate.

With these regards, literature proposes various tools and methods, such as for example :
- Pharmacogenetics, which consist in the study of individual response to drugs as a function of genetic differences. These responses relate to how a drug functions in any given individual, how it is metabolized, its toxicity and dosage requirements. With the human genome project, pharmacogenetics has expanded into pharmacogenomics. Pharmacogenomics goes beyond pharmacogenetics, with the potential to find uses from drug discovery and development, target discovery and validation, and clinical trials;
- Metabolomics can also be applied to the field of predictive medicine. Unlike pharmacogenetics, which is limited to genetic factors, pharmaco-metabolomics is able to predict an individual's response to a drug based not only on genetic factors, but also non-genetic factors, such as other drugs in the patient's body, the patient's current state of health, etc.
- The role of biomarkers is becoming increasingly important in the clinical development of therapeutics. A biomarker can be an indicator of normal biological processes, disease processes, or pharmacological responses to therapeutic intervention. Their role ranges from, stratifying the patient population in helping to identify responders versus non-responders to determining the efficacy of the therapeutic. Biomarkers can be a valuable tool in making better decisions that will reduce the cost for drug development and enable therapies to reach the right patient population faster.

Several publications describe the determination of levels of CD3+ and/or CD69+ T cells in patients in connection with different disease states or different treatments, e. g. Rossi et al. (Cancer Res. 65 (2005), 10555-10561), Ampel et al. (Mycopathologia 161 (2006), 67-72), Melichar et al. (Immunopharmacology and Immunotoxicology 23 (2001), 163-173), Devarapu et al. (Immunopharmacology and Immunotoxicology 28 (2006), 387-395), Lavasani et al. (Scandinavian J. Immunol. 65 (2007), 39-47) and Huang et al. (Pediatr. Allergy Immunol. 13 (2002), 426-433). However, none of them suggests a correlation of the levels of CD3+ CD69+ lymphocytes after administration of an immunogenic composition, in particular a composition comprising a recombinant viral vector, and the efficacy of a treatment with said composition.

The invention provides materials and methods for assessing the efficacy of a treatment involving the administration of an immunogenic composition to a patient (i.e. immunotherapy treatment) using biological markers (biomarkers) that have been determined to be substantially reliable signature which correlates with the desired therapeutic response, more particularly the desired immune response. The biomarkers are present in biological samples obtained from the patient. The ability to predict the clinical outcome of a treatment, soon after its initiation, will enable clinicians and patients to identify ineffective therapy, make informed decisions regarding the course of treatment, including whether to abandon or to allow alternate therapy implementation.

As used herein throughout the entire application, the terms "a" and "an" are used in the sense that they mean "at least one", "at least a first", "one or more" or "a plurality" of the referenced compounds or steps, unless the context dictates otherwise. For example, the term "a cell" includes a plurality of cells including a mixture thereof. More specifically, "at least one" and "one or more" means a number which is one or greater than one, with a special preference for one, two or three.

The term "and/or" wherever used herein includes the meaning of "and", "or" and "all or any other combination of the elements connected by said term".

The term "about" or "approximately" as used herein means within 20%, preferably within 10%, and more preferably within 5%.

The terms "patient", "subject" refer to a vertebrate, particularly a member of the mammalian species and includes, but is not limited to, domestic animals, sport animals, primates including humans.

As used herein, the term "treatment" or "treating" encompasses prophylaxis and/or therapy. Accordingly the immunogenic combinations or methods of the present invention are not limited to therapeutic applications and can be used in prophylaxis ones. This is covered by the term "to developing a prophylactic or therapeutic response, preferably immune response," herein. "Prophylaxis" is not limited to preventing immediate diseases (e.g. infectious diseases), it further encompasses prevention of long term consequences of these infections such as cirrhosis or cancer.

An "effective amount" or a "sufficient amount" of an active compound is an amount sufficient to effect beneficial or desired results, including clinical results. An effective amount can be administered in one or more administrations. A "therapeutically effective amount" is an amount to effect beneficial clinical results, including, but not limited to, alleviation of one or more symptoms associated with viral infection as well as prevention of disease (e.g. prevention of one or more symptoms of infection).

According to a first embodiment, the present invention ! relates to an ex-vivo method for assessing the efficacy of a treatment involving the administration of an immunogenic composition comprising at least one recombinant viral vector encoding a targeted antigen to a patient comprising:
- measuring levels of activated CD3+ CD69+ T lymphocytes in a biological sample taken from the body of said patient wherein one or more doses of said immunogenic composition had been administered to said patient and wherein said biological sample had been taken following at least one of the said administrations;
- wherein levels of activated CD3+ CD69+ T lymphocytes above about 10.4 % indicate that the subject is indicative of a successful clinical outcome for the treatment.

In a preferred embodiment a successful clinical outcome means an increase in survival rate.

Thus,invention provides an ex-vivo method for assessing the efficacy of a treatment involving the administration of an immunogenic composition to a patient (i.e. immunotherapy treatment).

According to the invention, the term "assessing" should be understood as "monitoring, modifying or adjusting" a treatment involving the administration of an immunogenic composition to a patient.

The method includes assessing the efficacy of immunotherapy treatment based on the level of activated CD3+ CD69+ T lymphocytes (in the following also referred to as "activated T lymphocytes (CD3+ CD69+)") in the patient following immunotherapy treatment.

The method includes measuring a patient's levels of activated T lymphocytes (CD3+ CD69+) following immunotherapy treatment; and assessing the efficacy of the immunotherapy treatment based on the levels of activated T lymphocytes (CD3+ CD69+).

In certain aspects, the method can further include measuring a patient's levels of activated T lymphocytes (CD3+ CD69+) prior to immunotherapy treatment.

In certain aspects, the method includes measuring a patient's levels of activated T lymphocytes (CD3+ CD69+) at least once several weeks following immunotherapy treatment; and assessing the efficacy of the immunotherapy treatment based on the levels of activated T lymphocytes (CD3+ CD69+).

The time between the initiation of immunotherapy treatment and activated T lymphocytes (CD3+ CD69+) measurements may be 1 day to about 48 weeks or more (e.g., from about 1 day to about 1 week, from about 1 week to about 2 weeks, from about 2 weeks to about 4 weeks, from about 4 weeks to about 8 weeks, from about 8 weeks to about 12 weeks, from about 12 weeks to about 16 weeks, from about 16 weeks to about 24 weeks, from about 24 weeks to about 48 weeks, or more). In a preferred embodiment of the invention, the time interval is about 5 weeks. Similarly, additional measurements (i.e., a third, fourth, fifth, etc. measurement) may be taken at similar time intervals following the second measurement.

The "immunotherapy treatment" consists in at least one administration of an immunogenic composition to a patient. According to special embodiment, the "immunotherapy treatment" consists in successive administrations of an immunogenic composition to a patient, more specifically it consists in weekly administration during at least 2 weeks, preferably during at least 6 weeks.

The method includes determining the levels of activated T lymphocytes (CD3+ CD69+) in a patient following administration of an immunogenic composition to the patient; comparing said levels to the above-mentioned cut-off value; and assessing the efficacy of immunotherapy treatment based on the levels of activated T lymphocytes (CD3+ CD69+) compared to the cut-off value.

As used herein, the terms "activated T lymphocytes (CD3+ CD69+)" means a lymphocyte cell which expresses CD3 and CD69 cell surface antigens.

According to the Invention, the levels of activated T lymphocytes (CD3+ CD69+) can be determined for example by flow cytometry (e.g. by flow cytofluorimetry), target cell lysis assay, and more particularly by 2 or more color flow cytometry (e.g. Beckton Dickinson, Beckman Coulter). See for example Chizzolini et al., 1991, Eur J Immunol.; 21(11),2727-33.

According to the Invention, the levels of activated T lymphocytes (CD3+ CD69+) can be determined on total blood sample or on isolated peripheral blood mononuclear cells (PBMC) [e.g. by Ficoll-Hypaque purification of peripheral blood mononuclear cells (PBMC) (Bennett & Breit 1994, J Leukoc Biol., 56(3), 236-40), or by using Sigma Accuspin™ system (Sigma-Aldrich Ltd.) according to the manufacturer's instructions, an the like].

According to one embodiment of the Invention, the level of activated T lymphocytes (CD3+ CD69+) is determined by using antibodies.

According to one specific embodiment of the Invention, said antibodies are monoclonal antibodies.

Examples of antibodies which could be used are anti-CD3 (Beckman Coulter Cat. No. A07748) and anti-CD69 (Beckman Coulter Cat. Nr. IM2656U)

According to one specific embodiment of the Invention, said antibodies are tagged for example by fluorescence, radiolabel, enzyme, biotin, or any other methods designed to render cells labelled with said antibodies detectable. These techniques are widely used and known in the art.

According to one preferred embodiment of the Invention, the levels of activated T lymphocytes (CD3+ CD69+) is determined by using antibodies specific for CD3 and CD69.

Thus, for example, the levels of activated T lymphocytes (CD3+ CD69+) is determined by collecting peripheral blood and incubating cells with monoclonal antibodies (e.g. with anti-CD3, and CD69). Then the levels of activated T lymphocytes (CD3+ CD69+) is determined with a flow cytometry instrument, for example as manufactured by Instrumentation Laboratory-Beckman Coulter, with a He-Ne laser-ray, which recognizes wavelengths of four different fluorochromes (fluorescein isothiocyanate FITC, phycoerythrin PE/RD1, ECD, PC5/PE).

The levels of activated T lymphocytes (CD3+ CD69+) can be expressed in either (i) percent (%) of peripheral blood lymphocytes which express CD3 and CD69 cell surface antigens, or (ii) absolute numbers of activated T lymphocytes (CD3+ CD69+) per microliter of whole peripheral blood.

Levels of activated T lymphocytes (CD3+ CD69+) of more than about 10.4% correspond to levels of more than 148 CD3+ CD69+ lymphocytes per µl whole blood. Adantageously said level of activated T lymphocytes (CD3+ CD69+) is determined at day 43 following initiation of the said treatment involving the administration of an immunogenic composition to a patient.

In a preferred embodiment of the invention, the method of the invention further comprises an initial step consisting in measuring the levels of activated T lymphocytes (CD3+ CD69+) in the body of the patient before administration of the immunogenic composition.

According to the present invention, the levels of activated T lymphocytes (CD3+ CD69+) is measured in a biological sample obtained from the patient. Biological samples include but are not limited to blood and other liquid samples of biological origin, solid tissue samples, such as a biopsy specimen. In a preferred embodiment, the biological sample is blood, plasma or serum, in which case obtaining the samples from a patient is relatively simple and non-invasive procedure. Methods of obtaining blood or serum are well-known in the art are not part of the invention.

As used herein, the terms "immunogenic composition" "vaccine composition", "vaccine" or similar terms can be used interchangeably and mean an agent suitable for stimulating/inducing/increasing a subject's immune system to ameliorate a current condition or to protect against or to reduce present or future harm or infections (including viral, bacterial, parasitic infections), e.g., reduced tumour cell proliferation or survival, reduced pathogen replication or spread in a subject or a detectably reduced unwanted symptom(s) associated with a condition, extend patient survival. Said immunogenic composition comprises at least one recombinant viral vector encoding a targeted antigen.

According to an alternate embodiment, the immunogenic composition of the Invention further comprises at least one immune response modifier.

Examples of such immune response modifiers (IRMs), include the CpG oligonucleotides (see US 6,194,388; US2006094683; WO 2004039829 for example), lipopolysaccharides, polyinosic:polycytidylic acid complexes (Kadowaki, et al., 2001, J. Immunol. 166, 2291-2295), and polypeptides and proteins known to induce cytokine production from dendritic cells and/or monocyte/macrophages. Other examples of such immune response modifiers (IRMs) are small organic molecule such as imidazoquinolinamines, imidazopyridine amines, 6,7-fused cycloalkylimidazopyridine amines, imidazonaphthyridine amines, oxazoloquinoline amines, thiazoloquinoline amines and 1,2-bridged imidazoquinoline amines (see for example US 4,689,338; US 5,389,640; US 6,110,929; and US 6,331,539).

As used herein, the term "antigen" refers to any substance, including complex antigen (e.g. tumour cells, virus infected cells, etc...), that is capable of being the target of an immune response. An antigen may be the target of, for example, a cell-mediated and/or humoral immune response raised by a patient. The term "antigen" encompasses for example all or part of viral antigens, tumour-specific or tumour-related antigens, bacterial antigens, parasitic antigens, and the like:
- Viral antigens include for example antigens from hepatitis viruses A, B, C, D and E, HIV, herpes viruses, cytomegalovirus, varicella zoster, papilloma viruses, Epstein Barr virus, influenza viruses, para-influenza viruses, adenoviruses, coxsakie viruses, picorna viruses, rotaviruses, respiratory syncytial viruses, pox viruses, rhinoviruses, rubella virus, papovirus, mumps virus, measles virus; some non-limiting examples of known viral antigens include the following : antigens derived from HIV-1 such as tat, nef, gp120 or gp160, gp40, p24, gag, env, vif, vpr, vpu, rev or part and/or combinations thereof; antigens derived from human herpes viruses such as gH, gL gM gB gC gK gE or gD or part and/or combinations thereof or Immediate Early protein such asICP27, ICP47, ICP4, ICP36 from HSV1 or HSV2 ; antigens derived from cytomegalovirus, especially human cytomegalovirus such as gB or derivatives thereof ; antigens derived from Epstein Barr virus such as gp350 or derivatives thereof; antigens derived from Varicella Zoster Virus such as gpl, 11, 111 and IE63; antigens derived from a hepatitis virus such as hepatitis B , hepatitis C or hepatitis E virus antigen (e.g. env protein E1 or E2, core protein, NS2, NS3, NS4a, NS4b, NS5a, NS5b, p7, or part and/or combinations thereof of HCV) ; antigens derived from human papilloma viruses (for example HPV6, 11, 16, 18, e.g. L1, L2, E1, E2, E3, E4, E5, E6, E7, or part and/or combinations thereof) ; antigens derived from other viral pathogens, such as Respiratory Syncytial virus (e.g. F and G proteins or derivatives thereof), parainfluenza virus, measles virus, mumps virus, flaviviruses (e. g. Yellow Fever Virus, Dengue Virus, Tick-borne encephalitis virus, Japanese Encephalitis Virus) or Influenza virus cells (e.g. HA, NP, NA, or M proteins, or part and/or combinations thereof);
- tumor-specific or -related antigens include but are not limited to, carcinoma, lymphoma, blastoma, sarcoma, and leukemia. More particular examples of such cancers include breast cancer, prostate cancer, colon cancer, squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, colorectal cancer, endometrial carcinoma, salivary gland carcinoma, kidney cancer, liver cancer, vulval cancer, thyroid cancer, hepatic carcinoma and various types of head and neck cancer, renal cancer, malignant melanoma, laryngeal cancer, prostate cancer. Cancer antigens are antigens which can potentially stimulate apparently tumor-specific immune responses. Some of these antigens are encoded, although not necessarily expressed, by normal cells. These antigens can be characterized as those which are normally silent (i.e., not expressed) in normal cells, those that are expressed only at low levels or at certain stages of differentiation and those that are temporally expressed such as embryonic and fetal antigens. Other cancer antigens are encoded by mutant cellular genes, such as oncogenes (e.g., activated ras oncogene), suppressor genes (e.g., mutant p53), fusion proteins resulting from internal deletions or chromosomal translocations. Still other cancer antigens can be encoded by viral genes such as those carried on RNA and DNA tumor viruses. Some non-limiting examples of tumor-specific or -related antigens include MART-1/Melan-A, gp100, Dipeptidyl peptidase IV (DPPIV), adenosine deaminase-binding protein (ADAbp), cyclophilin b, Colorectal associated antigen (CRC)-C017-1A/GA733, Carcinoembryonic Antigen (CEA) and its immunogenic epitopes CAP-1 and CAP-2, etv6, aml1, Prostate Specific Antigen (PSA) and its immunogenic epitopes PSA-1, PSA-2, and PSA-3, prostate-specific membrane antigen (PSMA), T-cell receptor/CD3-zeta chain, MAGE-family of tumor antigens (e.g., MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-82), MAGE-Xp3 (MAGE-B3), MAGE-Xp4 (MAGE-B4), MAGE-C1, MAGE-C2, MAGE-C3, MAGE-C4, MAGE-C5), GAGE-family of tumor antigens (e.g., GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family (e.g. MUC-1), HER2/neu, p21ras, RCAS1, alpha-fetoprotein, E-cadherin, alpha-catenin, beta-catenin and gamma-catenin, p120ctn, gp100.sup.Pmel117, PRAME, NY-ESO-1, cdc27, adenomatous polyposis coli protein (APC), fodrin, Connexin 37, Ig-idiotype, p15, gp75, GM2 and GD2 gangliosides, viral products such as human papilloma virus proteins, Smad family of tumor antigens, lmp-1, P1A, EBV-encoded nuclear antigen (EBNA)-1; brain glycogen phosphorylase, SSX-1, SSX-2 (HOM-MEL-40), SSX-1, SSX-4, SSX-5, SCP-1 and CT-7, and c-erbB-2;
- bacterial antigens includes for example antigens from Mycobacteria causing TB and leprosy, pneumocci, aerobic gram negative bacilli, mycoplasma, staphyloccocal infections, streptococcal infections, salmonellae, chlamydiae, neisseriae;
- other antigens includes for example antigens from malaria, leishmaniasis, trypanosomiasis, toxoplasmosis, schistosomiasis, filariasis.

Said antigen is encoded by a heterologous nucleotide sequence and is expressed in *vivo* by a recombinant viral vector.

In a particularly preferred embodiment the heterologous nucleotide sequence encodes one or more of all or part of the following antigens HBV-PreS1 PreS2 and Surface env proteins, core and polHIV-gp120 gp40,gp160, p24, gag, pol, env, vif, vpr, vpu, tat, rev, nef; HPV-E1, E2, E3, E4, E5, E6, E7, E8, L1, L2 (see for example WO 90/10459, WO 98/04705, WO 99/03885); HPV env protein E1 or E2, core protein, NS2, NS3, NS4a, NS4b, NS5a, NS5b, p7 (see for example WO2004111082, WO2005051420); Muc-1 (see for example US 5, 861, 381: US6, 054, 438; WO98/04727; WO98/37095).

According to variants of the invention, the immunogenic composition contains a recombinant viral vector encoding at least two antigens, i.e. containing a heterologous nucleotide sequence encoding at least two antigens, or at least two heterologous nucleotide sequences encoding at least two antigens.

According to another special embodiment, said heterologous nucleotide sequence encodes all or part of HPV antigen(s) selected in the group consisting of E6 early coding region of HPV, E7 early coding region of HPV and derivates or combination thereof.

The HPV antigen encoded by the recombinant viral vector, is selected in the group consisting of an HPV E6 polypeptide, an HPV E7 polypeptide or both an HPV E6 polypeptide and an HPV E7 polypeptide. The present invention encompasses the use of any HPV E6 polypeptide which binding to p53 is altered or at least significantly reduced and/or the use of any HPV E7 polypeptide which binding to Rb is altered or at least significantly reduced (Munger et al., 1989, EMBO J. 8, 4099-4105; Crook et al., 1991, Cell 67, 547-556; Heck et al., 1992, Proc. Natl. Acad. Sci. USA 89, 4442-4446; Phelps et al., 1992, J. Virol. 66, 2148-2427). A non-oncogenic HPV-16 E6 variant which is suitable for the purpose of the present invention is deleted of one or more amino acid residues located from approximately position 118 to approximately position 122 (+1 representing the first methionine residue of the native HPV-16 E6 polypeptide), with a special preference for the complete deletion of residues 118 to 122 (CPEEK). A non-oncogenic HPV-16 E7 variant which is suitable for the purpose of the present invention is deleted of one or more amino acid residues located from approximately position 21 to approximately position 26 (+1 representing the first amino acid of the native HPV-16 E7 polypeptide, with a special preference for the complete deletion of residues 21 to 26 (DLYCYE). According to a preferred embodiment, the one or more HPV-16 early polypeptide(s) in use in the invention is/are further modified so as to improve MHC class I and/or MHC class II presentation, and/or to stimulate anti-HPV immunity. HPV E6 and E7 polypeptides are nuclear proteins and it has been previously shown that membrane presentation permits to improve their therapeutic efficacy (see for example WO99/03885). Thus, it may be advisable to modify at least one of the HPV early polypeptide(s) so as to be anchored to the cell membrane. Membrane anchorage can be easily achieved by incorporating in the HPV early polypeptide a membrane-anchoring sequence and if the native polypeptide lacks it a secretory sequence (i.e. a signal peptide). Membrane-anchoring and secretory sequences are known in the art. Briefly, secretory sequences are present at the N-terminus of the membrane presented or secreted polypeptides and initiate their passage into the endoplasmic reticulum (ER). They usually comprise 15 to 35 essentially hydrophobic amino acids which are then removed by a specific ER-located endopeptidase to give the mature polypeptide. Membrane-anchoring sequences are usually highly hydrophobic in nature and serves to anchor the polypeptides in the cell membrane (see for example Branden and Tooze, 1991, in Introduction to Protein Structure p. 202-214, NY Garland).

The choice of the membrane-anchoring and secretory sequences which can be used in the context of the present invention is vast. They may be obtained from any membrane-anchored and/or secreted polypeptide comprising it (e.g. cellular or viral polypeptides) such as the rabies glycoprotein, of the HIV virus envelope glycoprotein or of the measles virus F protein or may be synthetic. The membrane anchoring and/or secretory sequences inserted in each of the early HPV-16 polypeptides used according to the invention may have a common or different origin. The preferred site of insertion of the secretory sequence is the N-terminus downstream of the codon for initiation of translation and that of the membrane-anchoring sequence is the C-terminus, for example immediately upstream of the stop codon.

The HPV E6 polypeptide in use in the present invention is preferably modified by insertion of the secretory and membrane-anchoring signals of the measles F protein. Optionally or in combination, the HPV E7 polypeptide in use in the present invention is preferably modified by insertion of the secretory and membrane-anchoring signals of the rabies glycoprotein.

The therapeutic efficacy of the recombinant viral vector can also be improved by using one or more nucleic acid encoding immunopotentiator polypeptide(s). For example, it may be advantageous to link the HPV early polypeptide(s) to a polypeptide such as calreticulin (Cheng et al., 2001, J. Clin. Invest. 108, 669-678), Mycobacterium tuberculosis heat shock protein 70 (HSP70) (Chen et al., 2000, Cancer Res. 60, 1035-1042), ubiquitin (Rodriguez et al., 1997, J. Virol. 71, 8497-8503) or the translocation domain of a bacterial toxin such as Pseudomonas aeruginosa exotoxin A (ETA(dIII)) (Hung et al., 2001 Cancer Res. 61, 3698-3703).

According to another embodiment the recombinant viral vector used in the invention comprises a nucleic acid encoding one or more early polypeptide(s) as above defined, and more particularly HPV-16 and/or HPV-18 early E6 and/or E7 polypeptides.

According to another special and preferred embodiment, said heterologous nucleotide sequence encodes all or part of MUC 1 antigen or derivates thereof.

According to another special embodiment, said heterologous nucleotide sequence encodes one or more of all or part of the followings: HCV env protein E1 or E2, core protein, NS2, NS3, NS4a, NS4b, NS5a, NS5b, p7 or derivates thereof. According to another special embodiment, said heterologous nucleotide sequence encodes one or more fusion protein wherein the configuration is not native in the sense that at least one of the NS polypeptides appears in an order which is distinct from that of the native configuration. Thus, if the fusion protein comprises a NS3 polypeptide, a NS4A polypeptide and a NS5B polypeptide, the native configuration would be NS3-NS4A-NS5B with NS3 at the N-terminus and NS5B at the C-terminus. In contrast, a non-native configuration can be NS5B-NS3-NS4A, NS5B-NS4A-NS3, NS4A-NS3-NS5B, NS4A-NS5B-NS3 or NS3-NS5B-NS4A. In particular, the fusion protein comprises at least one of the followings:
o A NS4A polypeptide fused directly or through a linker to the N-terminus of a NS3 polypeptide;
o A NS3 polypeptide fused directly or through a linker to the N-terminus of a NS5B polypeptide;
○ A NS4B polypeptide fused directly or through a linker to the N-terminus of a NS5B polypeptide;
o A NS4A polypeptide fused directly or through a linker to the N-terminus of a NS3 polypeptide which is fused directly or through a linker to the N-terminus of a NS4B polypeptide; and/or
o A NS3 polypeptide fused directly or through a linker to the N-terminus of a NS4B polypeptide which is fused directly or through a linker to the N-terminus of a NS5B polypeptide.

In such specific portions of the fusion protein each of the NS polypeptides can be independently native or modified. For example, the NS4A polypeptide included in the NS4A-NS3 portion can be native whereas the NS3 polypeptide comprises at least one of the modifications described below.

If needed, the nucleic acid molecule in use in the invention may be optimized for providing high level expression of the targeted antigen (e.g. HPV early polypeptide(s)) in a particular host cell or organism, e.g. a human host cell or organism. Typically, codon optimisation is performed by replacing one or more "native" (e.g. HPV) codon corresponding to a codon infrequently used in the mammalian host cell by one or more codon encoding the same amino acid which is more frequently used. This can be achieved by conventional mutagenesis or by chemical synthetic techniques (e.g. resulting in a synthetic nucleic acid). It is not necessary to replace all native codons corresponding to infrequently used codons since increased expression can be achieved even with partial replacement. Moreover, some deviations from strict adherence to optimised codon usage may be made to accommodate the introduction of restriction site(s).

As used herein, the term "recombinant viral vector" refers to extrachromosomal (e.g. episome), multicopy and integrating vectors (i.e. for being incorporated into the host chromosomes). Particularly important in the context of the invention are vectors for use in gene therapy (i.e. which are capable of delivering the nucleic acid to a host organism) as well as expression vectors for use in various expression systems.

Suitable viral vectors may be derived from a variety of different viruses (e.g. retrovirus, adenovirus, AAV, poxvirus, herpes virus, measle virus, foamy virus and the like). As used herein, the term "viral vector" encompasses vector DNA/RNA as well as viral particles generated thereof. Viral vectors can be replication-competent, or can be genetically disabled so as to be replication-defective or replication-impaired. The term "replication-competent" as used herein encompasses replication-selective and conditionally-replicative viral vectors which are engineered to replicate better or selectively in specific hosT lymphocytes (e.g. tumoral cells).

In one aspect, the recombinant viral vector in use in the invention is a recombinant adenoviral vector (for a review, see "Adenoviral vectors for gene therapy", 2002, Ed D. Curiel and J. Douglas, Academic Press). It can be derived from a variety of human or animal sources and any serotype can be employed from the adenovirus serotypes 1 through 51. Particularly preferred are human adenoviruses 2 (Ad2), 5 (Ad5), 6 (Ad6), 11 (Ad11), 24 (Ad24) and 35 (Ad35). Such adenovirus are available from the American Type Culture Collection (ATCC, Rockville, Md.), and have been the subject of numerous publications describing their sequence, organization and methods of producing, allowing the artisan to apply them (see for example US 6,133,028; US 6,110,735; WO 02/40665; WO 00/50573; EP 1016711; Vogels et al., 2003, J. Virol. 77, 8263-8271).

The adenoviral vector in use in the present invention can be replication-competent. Numerous examples of replication-competent adenoviral vectors are readily available to those skill in the art (see, for example, Hernandez-Alcoceba et al., 2000, Human Gene Ther. 11, 2009-2024; Nemunaitis et al., 2001, Gene Ther. 8, 746-759; Alemany et al., 2000, Nature Biotechnology 18, 723-727). For example, they can be engineered from a wild-type adenovirus genome by deletion in the E1A CR2 domain (see for example WO00/24408) and/or by replacement of the native E1 and/or E4 promoters with tissue, tumor or cell status-specific promoters (see for example US 5,998,205, WO99/25860, US 5,698,443, WO00/46355, WO00/15820 and WO01/36650).

Alternatively, the adenoviral vector in use in the invention is replication-defective (see for example WO94/28152; Lusky et al., 1998, J. Virol 72, 2022-2032). Preferred replication-defective adenoviral vectors are E1-defective (see for example US 6,136,594 and US 6,013,638), with an E1 deletion extending from approximately positions 459 to 3328 or from approximately positions 459 to 3510 (by reference to the sequence of the human adenovirus type 5 disclosed in the GeneBank under the accession number M 73260 and in Chroboczek et al., 1992, Virol. 186, 280-285). The cloning capacity can further be improved by deleting additional portion(s) of the adenoviral genome (all or part of the non essential E3 region or of other essential E2, E4 regions). Insertion of a nucleic acid in any location of the adenoviral vector can be performed through homologous recombination as described in Chartier et al. (1996, J. Virol. 70, 4805-4810). For example, the nucleic acid encoding the HPV-16 E6 polypeptide can be inserted in replacement of the E1 region and the nucleic acid encoding the HPV-16 E7 polypeptide in replacement of the E3 region or *vice versa.*

In another and preferred aspect, the vector in use in the invention is a poxviral vector (see for example Cox et al. in "Viruses in Human Gene Therapy" Ed J. M. Hos, Carolina Academic Press). According to another preferred embodiment it is selected in the group consisting of vaccinia virus, suitable vaccinia viruses include without limitation the Copenhagen strain (Goebel et al., 1990, Virol. 179, 247-266 and 517-563; Johnson et al., 1993, Virol. 196, 381-401), the Wyeth strain and the highly attenuated attenuated virus derived thereof including MVA (for review see Mayr, A., et al., 1975, Infection 3, 6-14) and derivates thereof (such as MVA vaccinia strain 575 (ECACC V00120707 - US 6,913,752), NYVAC (see WO 92/15672 - Tartaglia et al., 1992, Virology, 188, 217-232). Determination of the complete sequence of the MVA genome and comparison with the Copenhagen VV genome has allowed the precise identification of the seven deletions (I to VII) which occurred in the MVA genome (Antoine et al., 1998, Virology 244, 365-396), any of which can be used to insert the antigen-encoding nucleic acid. The vector may also be obtained from any other member of the poxviridae, in particular fowlpox (e.g. TROVAC, see Paoletti et al, 1995, Dev Biol Stand., 84, 159-163); canarypox (e.g. ALVAC, WO 95/27780, Paoletti et al, 1995, Dev Biol Stand., 84, 159-163); pigeonpox; swinepox and the like. By way of example, persons skilled in the art may refer to WO 92 15672 (incorporated by reference) which describes the production of expression vectors based on poxviruses capable of expressing such heterologous nucleotide sequence, especially nucleotide sequence encoding antigen.

The basic technique for inserting the nucleic acid and associated regulatory elements required for expression in a poxviral genome is described in numerous documents accessible to the man skilled in the art (Paul et al., 2002, Cancer gene Ther. 9, 470-477; Piccini et al., 1987, Methods of Enzymology 153, 545-563 ; US 4,769,330 ; US 4,772,848 ; US 4,603,112 ; US 5,100,587 and US 5,179,993). Usually, one proceed through homologous recombination between overlapping sequences (i.e. desired insertion site) present both in the viral genome and a plasmid carrying the nucleic acid to insert.

The nucleic acid encoding the antigen used in the present invention is preferably inserted in a nonessential locus of the poxviral genome, in order that the recombinant poxvirus remains viable and infectious. Nonessential regions are non-coding intergenic regions or any gene for which inactivation or deletion does not significantly impair viral growth, replication or infection. One may also envisage insertion in an essential viral locus provided that the defective function is supplied *in trans* during production of viral particles, for example by using an helper cell line carrying the complementing sequences corresponding to those deleted in the poxviral genome.

When using the Copenhagen vaccinia virus, the antigen-encoding nucleic acid is preferably inserted in the thymidine kinase gene (tk) (Hruby et al., 1983, Proc. Natl. Acad. Sci USA 80, 3411-3415 ; Weir et al., 1983, J. Virol. 46, 530-537). However, other insertion sites are also appropriate, e.g. in the hemagglutinin gene (Guo et al., 1989, J. Virol. 63, 4189-4198), in the K1L locus, in the u gene (Zhou et al., 1990, J. Gen. Virol. 71, 2185-2190) or at the left end of the vaccinia virus genome where a variety of spontaneous or engineered deletions have been reported in the literature (Altenburger et al., 1989, Archives Virol. 105, 15-27 ; Moss et al. 1981, J. Virol. 40, 387-395 ; Panicali et al., 1981, J. Virol. 37, 1000-1010 ; Perkus et al, 1989, J. Virol. 63, 3829-3836 ; Perkus et al, 1990, Virol. 179, 276-286 ; Perkus et al, 1991, Virol. 180, 406-410).

When using MVA, the antigen-encoding nucleic acid can be inserted in any one of the identified deletions I to VII as well as in the D4R locus, but insertion in deletion II or III is preferred (Meyer et al., 1991, J. Gen. Virol. 72, 1031-1038 ; Sutter et al., 1994, Vaccine 12, 1032-1040).

When using fowlpox virus, although insertion within the thymidine kinase gene may be considered, the antigen-encoding nucleic acid is preferably introduced in the intergenic region situated between ORFs 7 and 9 (see for example EP 314 569 and US 5,180,675).

According to a special embodiment, said recombinant viral vector is a recombinant adenoviral vector.

According to another special embodiment, said recombinant viral vector is a recombinant vaccinia vector.

According to one preferred embodiment, said recombinant vaccinia vector is a recombinant MVA vector.

Preferably, the antigen-encoding nucleic acid in use in the invention is in a form suitable for its expression in a host cell or organism, which means that the nucleic acid sequence encoding the antigen are placed under the control of one or more regulatory sequences necessary for its expression in the host cell or organism. As used herein, the term "regulatory sequence" refers to any sequence that allows, contributes or modulates the expression of a nucleic acid in a given host cell, including replication, duplication, transcription, splicing, translation, stability and/or transport of the nucleic acid or one of its derivative (i.e. mRNA) into the host cell. It will be appreciated by those skilled in the art that the choice of the regulatory sequences can depend on factors such as the host cell, the vector and the level of expression desired. The nucleic acid encoding the antigen is operatively linked to a gene expression sequence which directs the expression of the antigen nucleic acid within a eukaryotic cell. The gene expression sequence is any regulatory nucleotide sequence, such as a promoter sequence or promoter-enhancer combination, which facilitates the efficient transcription and translation of the antigen nucleic acid to which it is operatively linked. The gene expression sequence may, for example, be a mammalian or viral promoter, such as a constitutive or inducible promoter. Constitutive mammalian promoters include, but are not limited to, the promoters for the following genes: hypoxanthine phosphoribosyl transferase (HPRT), adenosine deaminase, pyruvate kinase, b-actin promoter and other constitutive promoters. Exemplary viral promoters which function constitutively in eukaryotic cells include, for example, promoters from the cytomegalovirus (CMV), simian virus (e.g., SV40), papilloma virus, adenovirus, human immunodeficiency virus (HIV), Rous sarcoma virus, cytomegalovirus, the long terminal repeats (LTR) of Moloney leukemia virus and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art. In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined antigen nucleic acid. The gene expression sequences optionally include enhancer sequences or upstream activator sequences as desired. Preferred promoters for use in a poxviral vector (see below) include without limitation vaccinia promoters 7.5K, H5R, TK, p28, p11 and K1L, chimeric promoters between early and late poxviral promoters as well as synthetic promoters such as those described in Chakrabarti et al. (1997, Biotechniques 23, 1094-1097), Hammond et al. (1997, J. Virological Methods 66, 135-138) and Kumar and Boyle (1990, Virology 179, 151-158).

The promoter is of special importance and the present invention encompasses the use of constitutive promoters which direct expression of the nucleic acid in many types of host cells and those which direct expression only in certain host cells or in response to specific events or exogenous factors (e.g. by temperature, nutrient additive, hormone or other ligand). Suitable promoters are widely described in literature and one may cite more specifically viral promoters such as RSV, SV40, CMV and MLP promoters. Preferred promoters for use in a poxviral vector include without limitation vaccinia promoters 7.5K, H5R, TK, p28, p11 and K1L, chimeric promoters between early and late poxviral promoters as well as synthetic promoters such as those described in Chakrabarti et al. (1997, Biotechniques 23, 1094-1097), Hammond et al. (1997, J. Virological Methods 66, 135-138) and Kumar and Boyle (1990, Virology 179, 151-158).

Those skilled in the art will appreciate that the regulatory elements controlling the expression of the nucleic acid molecule of the invention may further comprise additional elements for proper initiation, regulation and/or termination of transcription (e.g. polyA transcription termination sequences), mRNA transport (e.g., nuclear localization signal sequences), processing (e.g. splicing signals), and stability (e.g. introns and non-coding 5' and 3' sequences), translation (e.g. peptide signal, propeptide, tripartite leader sequences, ribosome binding sites, Shine-Dalgamo sequences, etc.) into the host cell or organism.

Alternatively, the recombinant viral vector in use in the present invention can further comprise at least one nucleic acid encoding at least one cytokine. Suitable cytokines include without limitation interleukins (e.g. IL-2, IL-7, IL-15, IL-18, IL-21) and interferons (e.g. IFNγ, INFα), with a special preference for interleukin IL-2. When the recombinant vaccine of the invention comprises a cytokine-expressing nucleic acid, said nucleic acid may be carried by the recombinant viral vector encoding the one or more antigen(s) or by an independent recombinant vector which can be of the same or a different origin.

According to one preferred embodiment, the recombinant viral vector in use in the present invention is encoding all or part of the MUC1 antigen or derivate thereof, and at least one cytokines above listed, and preferably an interleukin, especially IL2.

Infectious viral particles comprising the above-described recombinant viral vector can be produced by routine process. An exemplary process comprises the steps of:
a. introducing the viral vector into a suitable cell line,
b. culturing said cell line under suitable conditions so as to allow the production of said infectious viral particle,
c. recovering the produced infectious viral particle from the culture of said cell line, and
d. optionally purifying said recovered infectious viral particle.

Cells appropriate for propagating adenoviral vectors are for example 293 cells, PERC6 cells, HER96 cells, or cells as disclosed in WO 94/28152, WO 97/00326, US 6,127,175.

Cells appropriate for propagating poxvirus vectors are avian cells, and most preferably primary chicken embryo fibroblasts (CEF) prepared from chicken embryos obtained from fertilized eggs.

The infectious viral particles may be recovered from the culture supernatant or from the cells after lysis (e.g. by chemical means, freezing/thawing, osmotic shock, mecanic shock, sonication and the like). The viral particles can be isolated by consecutive rounds of plaque purification and then purified using the techniques of the art (chromatographic methods, ultracentrifugation on caesium chloride or sucrose gradient).

According to another embodiment, the methods of the invention may be combined with other methods for predicting the efficacy of treatment more specifically for predicting the efficacy of immunotherapy treatments. For example, levels of biomarkers such as levels of activated NK cells (see patent application claiming priority of EP 08305876.8) or levels of sICAM-1 (see patent application claiming priority of EP 09305032.6).

According to another preferred embodiment, the methods of the invention further comprise measuring the patient's levels of interferon γ as disclosed in patent application claiming priority of EP 09305256.1.

In certain aspects, the method thus includes measuring a patient's levels of interferon γ at least once several weeks following immunotherapy treatment; and assessing the efficacy of the immunotherapy treatment based on the levels of activated T lymphocytes (CD3+ CD69+) and of interferon γ.

The time between the initiation of immunotherapy treatment and interferon γ measurements may be 1 day to about 48 weeks or more (e.g., from about 1 day to about 1 week, from about 1 week to about 2 weeks, from about 2 weeks to about 4 weeks, from about 4 weeks to about 8 weeks, from about 8 weeks to about 12 weeks, from about 12 weeks to about 16 weeks, from about 16 weeks to about 24 weeks, from about 24 weeks to about 48 weeks, or more). In a preferred embodiment of the invention, the time interval is about 5 weeks. Similarly, additional measurements (i.e., a third, fourth, fifth, etc. measurement) may be taken at similar time intervals following the second measurement.

In related aspects the method includes determining the levels of interferon γ in a patient following administration of an immunogenic composition to the patient; comparing said levels to a cut-off value; and assessing the efficacy of immunotherapy treatment based on the levels of activated T lymphocytes (CD3+ CD69+) as described herein and on the levels of interferon γ compared to the cut-off value.

According to special embodiment of the Invention, "the levels of interferon γ" means detectable interferon γ level "detectable" being defined as ≥ to the limit of detection.

According to special embodiment, the cut-off value and/or limit of detection of interferon γ level are about 4 pg/ml (e.g. 4.6 pg/ml in plasma).

Advantageously said "levels of interferon γ" are determined at day 43 following initiation of the said treatment involving the administration of an immunogenic composition to a patient.

According to special embodiment, it has been shown a statistically significant correlation between patients who have detectable interferon γ level at day 43 and patients who have levels of CD3+ CD69+ lymphocytes above the above-indicated cut-off value at day 43 (Mann-Whitney, p = 0.02).

In a preferred embodiment of the invention, the method of the invention further comprises an initial step consisting in measuring the interferon γ levels in the body of the patient before administration of the immunogenic composition.

According to the present invention, the level of interferon γ is measured in a biological sample obtained from the patient. Biological samples include but are not limited to blood and other liquid samples of biological origin, solid tissue samples, such as a biopsy specimen. In a preferred embodiment, the biological sample is blood, plasma or serum, in which case obtaining the samples from a patient is relatively simple and non-invasive procedure. Methods of obtaining blood or serum are well-known in the art are not part of the invention.

In addition, numerous methods for detecting and quantifying polypeptides, including the instant biomarkers, are known. Such methods include but are not limited to antibody-based methods, more specifically monoclonal antibodies-based methods. The particular methods of detecting and quantifying the biomarkers are not important to the invention. For example the materials and methods of the present invention may be used with Luminex technology (Luminex Corporation, Austin, Tex.) or enzyme-linked immunosorbant assays (ELISA, numerous ELISA kits are commercially available e.g. by CliniScience, Diaclone, Biosource).

If desired, the administration of the immunogenic composition according to the Invention can be carried out in conjunction with one or more conventional therapeutic modalities (e.g. radiation, chemotherapy and/or surgery). The use of multiple therapeutic approaches provides the selected patient with a broader based intervention. In one embodiment, the administration of the immunogenic composition according to the Invention can be preceded or followed by a surgical intervention. In another embodiment, it can be preceded or followed by radiotherapy (e.g. gamma radiation). Those skilled in the art can readily formulate appropriate radiation therapy protocols and parameters which can be used (see for example Perez and Brady, 1992, Principles and Practice of Radiation Oncology, 2nd Ed. JB Lippincott Co; using appropriate adaptations and modifications as will be readily apparent to those skilled in the field). In still another embodiment, the administration of the immunogenic composition according to the Invention is associated to chemotherapy with one or more drugs (e.g. drugs which are conventionally used for treating or preventing viral infections, virus-associated pathologic conditions, cancer, and the like).

The method of the present invention can in particular be useful for improving the treatment of a cancer patient which is undergoing chemotherapeutic treatment with a chemotherapeutic agent.

According to one embodiment of such a method, the administration of said chemotherapeutic agent is done before administration of said immunogenic composition.

According to another embodiment of such a method, the administration of said chemotherapeutic agent is done after administration of said immunogenic composition.

According to another embodiment of such a method, the administration of said chemotherapeutic agent is done concomitantly with administration of said immunogenic composition.

In another embodiment, the method or use of the invention is carried out according to a prime boost therapeutic modality which comprises sequential administration of one or more primer composition(s) and one or more booster composition(s). Typically, the priming and the boosting compositions use different vehicles which comprise or encode at least an antigenic domain in common. The priming composition is initially administered to the host organism and the boosting composition is subsequently administered to the same host organism after a period varying from one day to twelve months. The method of the invention may comprise one to ten sequential administrations of the priming composition followed by one to ten sequential administrations of the boosting composition. Desirably, injection intervals are a matter of one week to six months. Moreover, the priming and boosting compositions can be administered at the same site or at alternative sites by the same route or by different routes of administration.

According to one special embodiment, the Invention relates to a method as above described wherein said human disease is cancer.

According to a special embodiment, said cancer is for example breast cancer, colon cancer, kidney cancer, rectal cancer, lung cancer, cancer of the head and neck, renal cancer, malignant melanoma, laryngeal cancer, ovarian cancer, cervical cancer, prostate cancer, non Small cell Lung Cancer (NSCLC), haematological cancers, gastric cancers, myeloma.

According to a preferred embodiment, said cancer is non Small cell Lung Cancer (NSCLC).

According to one special embodiment, the Invention relates to a method as above described wherein said human disease is infectious disease.

According to a preferred embodiment, said infectious disease is a viral induced disease, such as for example disease induced by HIV, HCV, HBV, HPV, and the like.

According to one special embodiment, the immune response observed in the treated patient population is directed towards a tumour-specific or -related antigens and/or viral antigen. According to one embodiment, said "immune response" in said patient population is directed towards distinct antigens. According to one special embodiment, said "immune response" in said patient population is directed towards MUC1 antigen. According to another special embodiment, said "immune response" in said patient population is T cell immune response, and preferably CD8+ (Cytotoxic T Lymphocytes) immune response. According to another special embodiment, said "immune response" in said patient population is a non specific immune response. According to another special embodiment, said "immune response" in said patient population is a stimulation of the innate immune response.

The ability to induce or stimulate an immune response upon administration in an animal or human organism can be evaluated either *in vitro* or *in vivo* using a variety of assays which are standard in the art. For a general description of techniques available to evaluate the onset and activation of an immune response, see for example Coligan et al. (1992 and 1994, Current Protocols in Immunology ; ed J Wiley & Sons Inc, National Institute of Health). Measurement of cellular immunity can be performed by measurement of cytokine profiles secreted by activated effector cells including those derived from CD4+ and CD8+ T-cells (e.g. quantification of IL-10 or IFN gamma-producing cells by ELIspot), by determination of the activation status of immune effector cells (e.g. T cell proliferation assays by a classical [³H] thymidine uptake), by assaying for antigen-specific T lymphocytes in a sensitized subject (e.g. peptide-specific lysis in a cytotoxicity assay) or by detection of antigen specific T lymphocytes by fluorescent MHC and/or peptide multimers (e.g. tetramers). The ability to stimulate a humoral response may be determined by antibody binding and/or competition in binding (see for example Harlow, 1989, Antibodies, Cold Spring Harbor Press). The method of the invention can also be further validated in animal models challenged with an appropriate tumor-inducing agent (e.g. MUC1-expressing TC1 cells) to determine anti-tumor activity, reflecting an induction or an enhancement of an anti-antigen immune response.

The method of the present invention can also be useful for extending the survival of a patient treated for human disease by administering an immunogenic composition.

Also disclosed is the use of levels of activated T lymphocytes (CD3+ CD69+) as a biomarker for predicting whether a subject treated by administration of an immunogenic composition comprising at least one recombinant viral vector encoding a targeted antigen is or is not susceptible to developing prophylactic or therapeutic response, preferably prophylactic or therapeutic immune response as a follow up to the said administration of an immunogenic composition.

According to another embodiment, the present Invention relates to the use of levels of activated T lymphocytes (CD3+ CD69+) as a biomarker for monitoring, modifying or adjusting a treatment involving the administration of an immunogenic composition comprising at least one recombinant viral vector encoding a targeted antigen to a patient, wherein levels of activated CD3+ CD69+ T lymphocytes above about 10.4% indicate that the subject is indicative of a successful clinical outcome for the treatment.

More specifically, the present Invention relates to the use of the level of activated T lymphocytes (CD33+ CD69+) and/or of levels of activated T lymphocytes (CD3+ CD69+) as a biomarker for monitoring, modifying or adjusting a treatment involving the administration of an immunogenic composition comprising at least one recombinant viral vector encoding a targeted antigen to a patient, wherein levels of activated T lymphocytes (CD3+ CD69+) measured after said administration above about 10.4% indicate that the subject is indicative of a successful clinical outcome for the treatment, i.e., the increase in survival rate.

Also described are kits which include parts for practicing the methods described herein and that will be apparent from the examples provided herein. The kit of parts, or kits, may include reagents for collecting and or measuring serum levels of interferon gamma. Such reagents may include antibodies. The kits may further include equipment for collecting and/or processing biological samples. The kits are also likely to contain instructions for use, cut-off values and/or instructions for their determination, and instructions for interpreting the data obtained from the use of the kits.

The said kit of parts, or kits, may further include an immunogenic composition as above disclosed, and/or as disclosed in the Example section below.

Also described are computer programs and/or algorithms for monitoring clinical trial and levels of activated T lymphocytes (CD3+ CD69+) (and eventually other biomarkers levels, such as for example interferon gamma levels), determining whether such levels are above or below a threshold level, and/or recommending modifications to a treatment regiment to improve a patient's response to an immunotherapy treatment. The computer programs or algorithms may be provided along with necessary hardware, e.g., in the form of a kit or apparatus, which may also accept biological samples and measure the relative levels of activated T lymphocytes (CD3+ CD69+) (and eventually other biomarkers levels, such as for example interferon gamma levels). The above-described computer programs and/or apparatus are likely to be provided to physicians or clinical laboratories with appropriate instructions and reagents, including antibodies.

The invention has been described in an illustrative nanner, and it is to be understood that the terminology which has been used is intended to be in the nature of words of description rather than of limitation. Obviously, many modifications and variations of the present invention are possible in light of the above teachings. It is therefore to be understood that within the scope of the appended claims, the invention may be practiced in a different way from what is specifically described herein.

### Figures :

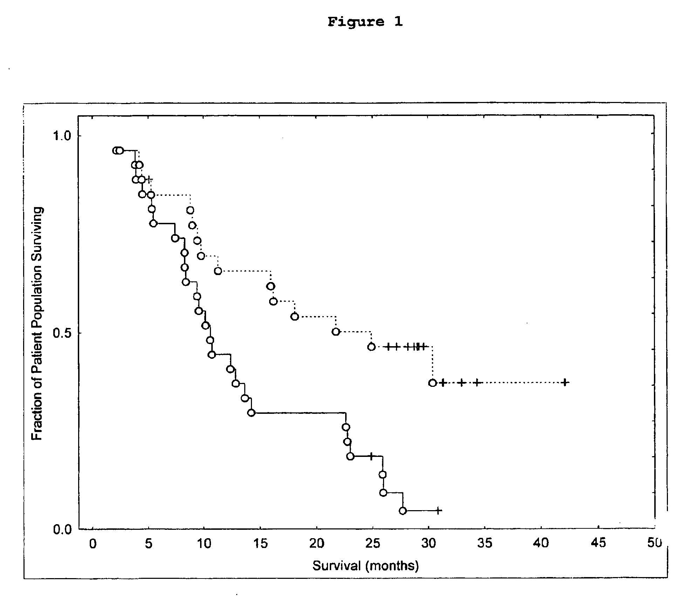

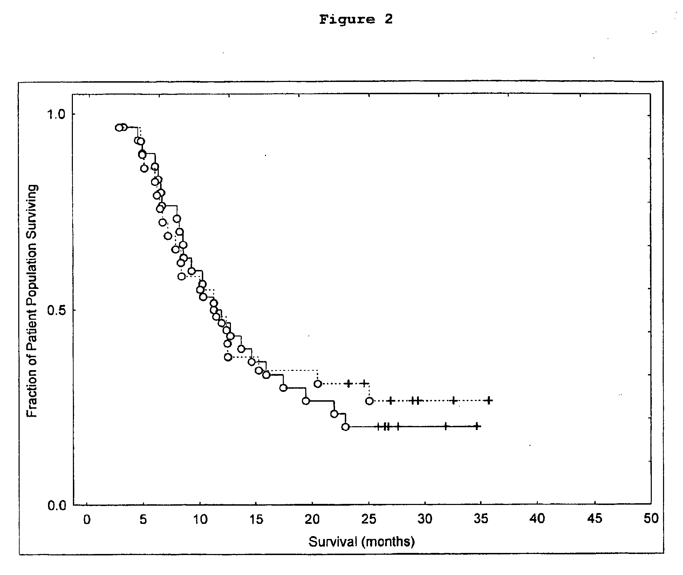

**Figure 1****:** Survival curves describing vaccine immunotherapy in lung cancer: Therapeutic vaccine (i.e. immunogenic composition) + chemotherapy in patients with above or blelow median levels of CD3+ CD69+ lymphocytes Day 43. Median level of CD3+ CD69+ lymphocytes for the study at Day 43 was 15 10.4 %.
   - - - Group 1: Therapeutic vaccine (i.e. immunogenic composition) + chemotherapy in patients with above median levels of CD3+ CD69+ lymphocytes Day 43. Overall patient survival for this group was = 21.9 months. 28 patients
   _______ Group 2: Therapeutic vaccine (i.e. immunogenic composition) + chemotherapy in patients with below median levels of CD3+ CD69+ lymphocytes Day 43. Median survival = 10.4 months. 29 patients.
   Significant difference (by long rank), group 1 and group 2 by log rank: p = 0.005
   ○ Complete + Censored
**Figure 2****:** Survival curves describing chemotherapy in lung cancer: in patients with above or blelow median levels of CD3+ CD69+ lymphocytes Day 43. Median level of CD3+ CD69+ lymphocytes for the study at Day 43 was 10.4 %.
   - - - Group 1: Chemotherapy in patients with above median levels of CD3+ CD69+ lymphocytes Day 43. Overall patient survival for this group = 11.4 months. 28 patients
   _______Group 2: Chemotherapy in patients with below median levels of CD3+ CD69+ lymphocytes Day 43. Median survival = 11.3 months. 29 patients.
   Median survival between the two groups of patients is not significantly different (by long rank), group 1 and group 2 by log rank: p = 0.79
   ○ Complete + Censored

### EXAMPLES

In the clinical study TG4010.09 (MVA-MUC1-IL2 in late stage Non Small Cell Lung Cancer) the cancer immunotherapeutic TG4010 was tested in combination with standard chemotherapy and compared to chemotherapy alone. Analysis of the lymphocyte phenotype, by flow cytometry, after initiation of therapy (day 43, one week following the 6^{th} injection of TG4010) showed heterogeneity in levels of activated T lymphocytes (CD3+ CD69+). Comparisons of levels of CD3+ CD69+ lymphocytes and patient survival by Spearman correlation coefficient showed a significant correlation between patient levels of CD3+ CD69+ lymphocytes at day 43 and survival, only in patients treated with TG4010 + chemotherapy, not in patients treated with chemotherapy alone. Kaplan-Meier plots survival of patient survival according to levels of CD3+ CD69+ lymphocytes show a significant association between survival of patients treated with TG4010 + chemotherapy and levels of CD3+ CD69+ lymphocytes at Day 43.

### Example 1 :

The immunogenic composition, noted vaccine TG4010, was used to treat non-small cell lung cancer (NSCLC) patients in combination with standard chemotherapy.

TG4010 is a recombinant Modified Virus Ankara (MVA) expressing both IL2 and the tumor-associated antigen MUC1.

One hundred and forty eight patients were randomized to receive:
- chemotherapy (Cisplatin 75mg/m² on dl and Gemcitabine 1250mg/m² on day 1 and day 8 every 3 weeks for up to 6 cycles) either alone (Study Arm 2) or
- chemotherapy together with TG4010 (Study Arm 1).

Tumors were evaluated (WHO criteria) every 6 weeks. Endpoints were progression-free survival (PFS) at 6 months and overall survival with intent to treat analysis.

Blood samples were taken at day 43 (one week following the 6^{th} weekly injection) and were shipped immediately to a central immunology lab where Peripheral Blood Mononuclear Cells (PBMC) were purified and stored frozen until analysis in batch.

PBMC samples were assessed for content of lymphocyte subsets by 5 color flow cytometry, using antibodies specific for lymphocyte CD markers CD3, CD8, CD16, CD56 and CD69.

Examples of antibodies which could be used are anti-CD3 (Beckman Coulter Cat. No. A07748), anti-CD8 (Beckman Coulter Cat. No. 6607102), anti-CD16 (Beckman Coulter Cat. Nr. IM0814U), anti-CD56 (Beckman Coulter Cat. Nr. A07788U) and anti-CD69 (Beckman Coulter Cat. Nr. IM2656U).

Figure 1 shows that patients [Arm 1 (TG4010 -+ chemotherapy] with above median levels of activated T lymphocytes (identified by the markers CD3+ CD69+ at day 43) survive longer (median survival = 21.9 months) than do patients with below median levels of CD3+ CD69+ lymphocytes (median survival 10.4 months) when treated with both the TG4010 vaccine and chemotherapy.

The data in Figure 2 demonstrate that the positive association between higher than median levels of CD3+ CD69+ lymphocytes at day 43 and overall survival is restricted to patients receiving the therapeutic vaccine. Patients in arm 2, receiving chemotherapy alone, who had above median levels of CD3+ CD69+ lymphocytes at day 43 did not survive any longer than patients with below median levels of CD3+ CD69+ lymphocytes at day 43 (11.4 months vs 11.3 months respectively.

## Claims

1. An ex-vivo method for assessing the efficacy of a treatment involving the administration of an immunogenic composition comprising at least one recombinant viral vector encoding a targeted antigen to a patient comprising:
- measuring levels of activated CD3+ CD69+ T lymphocytes in a biological sample taken from the body of said patient wherein one or more doses of said immunogenic composition had been administered to said patient and wherein said biological sample had been taken following at least one of the said administrations;
- wherein levels of activated CD3+ CD69+ T lymphocytes above about 10.4 % indicate that the subject is indicative of a successful clinical outcome for the treatment.

2. The method of claim 1, wherein the successful clinical outcome is the increase in survival rate.

3. The method of claim 1 or 2 wherein said immunogenic composition further comprises at least one immune response modifier.

4. The method of any of the previous claims wherein said patient is suffering of cancer.

5. The method of claim 4 wherein said cancer is Non Small Cell Lung Cancer or kidney cancer.

6. The method of any one of claims 1 to 5 wherein said targeted antigen is a tumour specific antigen.

7. The method of claim 6 wherein said tumour specific antigen is MUC1.

8. Use of levels of activated CD3+ CD69+ T lymphocytes as a biomarker for monitoring, modifying or adjusting a treatment involving the administration of an immunogenic composition comprising at least one recombinant viral vector encoding a targeted antigen to a patient, wherein levels of activated CD3+ CD69+ T lymphocytes above about 10.4 % indicate that the subject is indicative of a successful clinical outcome for the treatment.

9. The use of claim 8, wherein said successful clinical outcome is the increase in survival rate.

## Patentansprüche

1. Ex-vivo-Verfahren zur Effizienzbewertung einer Behandlung, die die Gabe einer immunogenen Zusammensetzung an einen Patienten beinhaltet, umfassend mindestens einen rekombinanten viralen Vektor, der ein Zielantigen codiert, umfassend:
- Messung des Spiegels von aktivierten CD3⁺-CD69⁺-T-Lymphocyten in einer biologischen Probe, die aus dem Körper des Patienten genommen wurde, wobei eine oder mehrere Dosen der immunogenen Zusammensetzung an den Patienten verabreicht worden waren und wobei die biologische Probe nach mindestens einer der Gaben entnommen worden war;
- wobei Spiegel der aktivierten CD3⁺-CD69⁺-T-Lymphocyten von über etwa 10.4% anzeigen, dass das Individuum ein Indikator für ein erfolgreiches klinisches Ergebnis der Behandlung ist.

2. Verfahren nach Anspruch 1, wobei das erfolgreiche klinische Ergebnis die Zunahme der Überlebensrate ist.

3. Verfahren nach Anspruch 1 oder 2, wobei die immunogene Zusammensetzung außerdem mindestens einen Immunantwortmodifikator umfasst.

4. Verfahren nach einem der vorherigen Ansprüche, wobei der Patient an Krebs leidet.

5. Verfahren nach Anspruch 4, wobei der Krebs nichtkleinzelliges Bronchialkarzinom oder Nierenkrebs ist.

6. Verfahren nach einem der Ansprüche 1 bis 5, wobei das angezielte Antigen ein Tumor-spezifisches Antigen ist.

7. Verfahren nach Anspruch 6, wobei das Tumor-spezifische Antigen MUC1 ist.

8. Verwendung der Spiegel von aktivierten CD3⁺-CD69⁺-T-Lymphocyten als Biomarker zur Beobachtung, Modifikation oder Anpassung einer Behandlung, die die Gabe einer immunogenen Zusammensetzung an einen Patienten umfasst, die mindestens einen rekombinanten viralen Vektor umfasst, der ein angezieltes Antigen codiert, wobei Spiegel von aktivierten CD3⁺-CD69⁺-T-Lymphocyten von über etwa 10.4% anzeigen, dass das Individuum ein Indikator für ein erfolgreiches klinisches Ergebnis der Behandlung ist.

9. Verwendung nach Anspruch 8, wobei das erfolgreiche klinische Ergebnis die Zunahme der Überlebensrate ist.

## Revendications

1. Méthode ex vivo pour évaluer l'efficacité d'un traitement impliquant l'administration d'une composition immunogène comprenant au moins un vecteur viral recombinant codant pour un antigène cible à un patient comprenant :
- la mesure des niveaux des lymphocytes T CD3+ CD69+ activés dans un échantillon biologique prélevé à partir du corps dudit patient dans laquelle une ou plusieurs doses de ladite composition immunogène a été administrée audit patient et dans laquelle ledit échantillon biologique a été pris à la suite d'au moins une desdites administrations ;
- dans laquelle les niveaux des lymphocytes T CD3+ CD69+ au-dessus d'environ 10,4% indiquent que le sujet est en voie d'une issue clinique favorable pour le traitement.

2. Méthode selon la revendication 1, dans laquelle l'issue clinique favorable est une augmentation du taux de survie.

3. Méthode selon la revendication 1 ou 2 dans laquelle ladite composition immunogéne comprend en outre au moins un modificateur de la réponse immune.

4. Méthode selon quelconque des revendications précédentes dans laquelle ledit patient souffre d'un cancer.

5. Méthode selon la revendication 4 dans laquelle ledit cancer est un Cancer du Poumon Non à Petites Cellules ou un cancer du rein.

6. Méthode selon l'une quelconque des revendications 1 à 5 dans laquelle ledit antigène cible est un antigène tumeur spécifique.

7. Méthode selon la revendication 6 dans laquelle l'antigène tumeur spécifique est MUC1.

8. Utilisation des niveaux des lymphocytes T CD3+ CD69+ activés en tant que biomarqueur pour suivre, modifier ou ajuster un traitement impliquant l'administration d'une composition immunogène comprenant au moins un vecteur viral recombinant codant pour un antigène cible à un patient, dans laquelle les niveaux des lymphocytes T CD3+ CD69+ au-dessus d'environ 10,4% indiquent que le sujet est en voie d'une issue clinique favorable pour le traitement.

9. Utilisation selon la revendication 8, dans laquelle l'issue clinique favorable est une augmentation du taux de survie.
